(19) Europäisches Patentamt
European Patent Office
Office européen des brevets

(11) Numéro de publication : **0 482 979 A1**

(12) **DEMANDE DE BREVET EUROPEEN**

(21) Numéro de dépôt : **91402737.0**

(22) Date de dépôt : **14.10.91**

(51) Int. Cl.$^5$ : **C07C 45/51,** C07C 47/575, C07C 47/565, C07C 47/542, C07C 47/21

(30) Priorité : **26.10.90 FR 9013269**

(43) Date de publication de la demande : **29.04.92 Bulletin 92/18**

(84) Etats contractants désignés : **AT BE CH DE DK ES FR GB GR IT LI LU NL SE**

(71) Demandeur : **RHONE-POULENC CHIMIE
25, quai Paul Doumer
F-92408 Courbevoie Cédex (FR)**

(72) Inventeur : **Bonneau, Isabelle, Résidence Charrière Blanche
Chemin de Charrière Blanche
F-69130 Ecully (FR)**
Inventeur : **Gubelmann, Michel
105, rue de Créqui
F-69006 Lyon (FR)**
Inventeur : **Tirel, Philippe-Jean
40, boulevard Kennedy
F-69600 Oullins (FR)**

(74) Mandataire : **Dutruc-Rosset, Marie-Claude et al
RHONE-POULENC CHIMIE Service Brevets Chimie 25, Quai Paul Doumer
F-92408 Courbevoie Cédex (FR)**

(54) **Procédé de préparation d'aldéhydes à partir d'alcools secondaires alpha-trihalogénés.**

(57) La présente invention a pour objet un procédé de préparation d'aldéhydes à partir d'alcools secondaires α-trihalogénés.

La caractéristique du procédé de l'invention consiste à effectuer la réaction de scission de l'alcool secondaire α-trihalogéné en aldéhyde et trihalogénométhane correspondants dans un solvant peu ou moyennement polaire et en présence d'un catalyseur gui est un sel à propriétés basiques, insoluble dans le milieu réactionnel.

EP 0 482 979 A1

La présente invention a pour objet un procédé de préparation d'aldéhydes à partir d'alcools secondaires $\alpha$-trihalogénés ou trihalo-génométhylcarbinols.

Les alcools secondaires $\alpha$-trihalogénés sont des produits connus et différents procédés de transformation de ces composés, soit en $\alpha$-hydroxyacides, soit en aldéhydes sont décrits dans l'état de la technique.

Le brevet FR-A 583856 de AKTIENGESELLSCHAFT FUR ANILIN FABRIKATION décrit un procédé de préparation de la vanilline (méthoxy-3 hydroxy-4 benzaldéhyde) à partir du méthoxy-3 hydroxy-4 phényltrichlorométhylcarbinol par chauffage à reflux en solution aqueuse en présence de grosses quantités d'acétate de cuivre.

Une telle préparation est longue (12 heures en général) et nécessite des quantités prohibitives d'acétate de cuivre, ce qui rend le procédé coûteux et non transposable à l'échelle industrielle. En outre, on opère avec des dilutions importantes.

Le brevet FR-A 727165 de I.G. FARBENINDUSTRIE, décrit un autre procédé de scission des méthoxy-3 hydroxy-4 phényltrichlorométhylcarbinol et éthoxy-3 hydroxy-4 phényltrichlorométhylcarbinol conduisant aux aldéhydes correspondants.Ledit procédé consiste à traiter lesdits phényltrichlorométhylcarbinols par un alcoolate de métal alcalin ou un hydrate alcoolique de métal alcalin ou alcalino-terreux, dans le méthanol, sous reflux. On obtient ainsi des rendements d'environ 50 à 75 % en aldéhydes, mais l'isolement de ce produit nécessite une longue succession d'opérations : traitement par un acide pour libérer l'aldéhyde obtenu sous forme de phénate, élimination du solvant, extraction par du chloroforme, puis rectification de l'aldéhyde.

On a également proposé selon le brevet FR-A 791818 de RHONE-POULENC et ses certificats d'addition FR-A 46789 et FR-A 47291, un procédé de préparation d'aldéhydes à partir d'aryltrichlorométhylcarbinols qui consiste à effectuer la saponification et l'oxydation d'aryltrichlorométhylcarbinols par une solution aqueuse alcaline à ébullition (ou à température plus élevée) de chromate de métal alcalin ou alcalino-terreux, de bioxyde de manganèse, de bioxyde de plomb, de peroxyde de baryum, d'eau oxygénée, de perborate ou de persulfate alcalins, éventuellement en présence de cuivre métallique.

Selon ce procédé, il faut opérer en milieu très dilué et il se forme des sous-produits de la réaction, qu'il faut ensuite séparer du milieu réactionnel.

Un autre procédé mentionné dans le brevet GB-A 453 482 de MONSANTO CHEMICALS LIMITED consiste à préparer des alcoxy- hydroxybenzaldéhydes par oxydation de l'acide alcoxy- hydroxy- phénylglycolique correspondant effectuée par chauffage dans une solution aqueuse d'hydroxyde alcalin en présence d'un composé aromatique nitré tel que le nitrobenzène, le m-dinitrobenzène ou le m-nitrotoluène. Ledit acide glycolique est obtenu par hydrolyse du composé phényltrichlorométhylcarbinol correspondant.

Plus récemment, le brevet FR-A 2244743 de SHELL INTERNATIONALE RESEARCH MAATSCHAPPIJ B.V. décrit la synthèse d'hydroxybenzaldéhydes, par scission de trichlorométhylcarbinols, dans des solvants aprotiques polaires, en présence d'une base choisie parmi les hydrures, alcoolates ou carbonates d'un métal alcalin ou alcalino-terreux. La durée de réaction demeure assez élevée (4 à 24 heures). Le traitement de récupération des aldéhydes obtenus n'est pas aisé, d'autant plus que l'acidification nécessaire en milieu aqueux engendre les difficultés de séparation ultérieure du solvant aprotique polaire et de l'eau, si l'on désire ne pas perdre des quantités importantes de ces coûteux solvants. La demande de brevet EP-A 44009 de BAYER décrit à titre de nouveaux composés, les [hydroxy-3 (trichloro-2,2,2 hydroxy-1 éthyl)-4] phényl-1 triazole-1,2,3 et indique qu'ils peuvent être transformés en aldéhydes correspondants par action d'un hydroxyde ou d'un alcoolate de métal alcalin ou alcalino-terreux, en milieu aprotique polaire.

L'opération d'isolement de l'aldéhyde obtenu, du milieu réactionnel pose les mêmes problèmes que pour le procédé précédent.

Une caractéristique commune à tous ces procédés est qu'ils ne sont pas adaptés à d'autres composés que les dérivés hydroxyphényltrichlorométhylcarbinols. Il semble que la présence de la fonction phénol soit nécessaire. En outre, les opérations de séparation des aldéhydes obtenus à partir du milieu réactionnel, sont relativement longues et complexes.

Un objet de l'invention est donc de fournir un procédé très général d'obtention d'aldéhydes à partir d'alcools secondaires $\alpha$-trihalogénés répondant à la formule générale (I)

$$Q{-}\underset{\displaystyle OH}{\underset{|}{CH}}{-}CX_3 \qquad (I)$$

dans ladite formule, Q représente un radical hydrocarboné monovalent, éventuellement substitué ayant de 1 à 40 atomes de carbone et X symbolise un atome d'halogène, chlore, fluor, brome, iode et, de préférence, le

chlore.

Dans la formule (I), le groupe

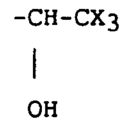

sera appelé "trihalogénométhylcarbinol".

La caractéristique du procédé de l'invention consiste à effectuer la scission des alcools secondaires α-trihalogénés en aldéhydes et trihalogénométhane correspondants dans un solvant aprotique peu ou moyennement polaire et en présence d'un catalyseur qui est un sel à propriétés basiques, insoluble dans le milieu réactionnel.

Dans l'exposé qui suit de la présente invention, on entend par solvant aprotique peu ou moyennement polaire, un solvant qui ne contient pas " de groupements de type $G_\alpha$ X H", dans ladite formule :

- G symbolise un radical hydrocarboné aliphatique saturé ou insaturé ayant de 1 à 4 atomes de carbone ou un radical cyclique saturé, insaturé ou aromatique ayant 5 ou 6 atomes,
- X représente O, S, COO, CSS, COS, $SO_3$, $PO_3$ lorsque $\alpha = 1$ et X représente N, P lorsque $\alpha = 2$.

On désigne également par sel à propriétés basiques, un sel dont l'anion a un $pK_b$ en milieu aqueux à 25°C compris entre 0 et 14, et de préférence entre 0 et 11, et encore plus préférentiellement compris entre 0 et 8. On définit le $pK_b$ selon l'équation bien connue : $pK_b = 14 - pK_a$ , le $pK_a$ étant le cologarithme de la constante de dissociation de l'acide en milieu aqueux.

Conformément au procédé de l'invention, la réaction de scission de l'alcool secondaire α-trihalogéné répondant à la formule (I) a lieu selon l'équation générale :

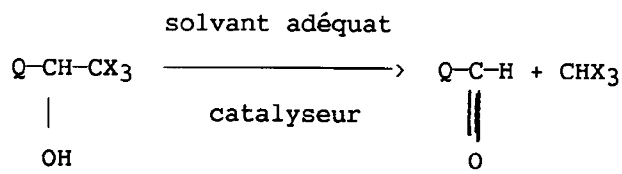


Le procédé de l'invention est caractérisé par le fait que la réaction de scission du trihalogénométhylcarbinol a lieu en système catalytique hétérogène, solide-liquide. Le catalyseur est en suspension dans une phase liquide comprenant le trihalogénométhylcarbinol et le solvant organique.

Un tel système catalytique permet d'obtenir un bon taux de conversion du trihalogénométhylcarbinol et une très bonne sélectivité de la réaction. Il en résulte que l'aldéhyde est obtenu avec un excellent rendement.

Un autre avantage du procédé de l'invention est la facilité de récupération de l'aldéhyde formé et du catalyseur du fait que la réaction a lieu en phase hétérogène.

Enfin, un autre aspect positif du procédé de l'invention est qu'il permet de préparer un aldéhyde, sans effluent polluant car le trihalogénométhane formé peut être valorisé.

Plus précisément, les alcools secondaires α-trihalogénés ou trihalogénométhylcarbinols servant de produits de départ à la préparation des aldéhydes répondent à la formule générale (I) dans laquelle Q représente un radical hydrocarboné monovalent, substitué ou non qui peut être un radical aliphatique acyclique saturé ou insaturé, linéaire ou ramifié ; un radical carboxyclique ou hétérocyclique saturé, insaturé ou aromatique, monocyclique ou polycyclique.

Conviennent tout particulièrement à la mise en oeuvre du procédé de l'invention, les trihalogénométhylcarbinols de formule générale (I) dans laquelle Q représente un radical hydrocarboné aromatique monocyclique ou polycyclique ; les radicaux pouvant former entre eux des systèmes orthocondensés, ortho- et péricondensés. On peut citer plus particulièrement le radical naphtyle.

Q représente préférentiellement un radical aryle répondant à la formule générale (II) :

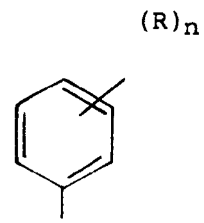

(II)

dans ladite formule (II):

– n est un nombre entier de 0 à 5, de préférence de 0 à 3,

– R représente $R_1$, l'un des groupes ou fonctions suivantes:

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,

. un radical alcényle linéaire ou ramifié ayant de 2 à 6 atomes de carbone, de préférence, de 2 à 4 atomes de carbone, tel que vinyle, allyle,

. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,

. un radical de formule

$-R_2-OH$
$-R_2-COOH$
$-R_2-CHO$
$-R_2-NO_2$
$-R_2-CN$
$-R_2-NH_2$
$-R_2-SH$
$-R_2-X$
$-R_2-CF_3$

dans lesdites formules $R_2$ représente un lien valentiel ou un radical hydrocarboné divalent, linéaire ou ramifié, saturé ou insaturé, ayant de 1 à 4 atomes de carbone tel que par exemple, méthylène éthylène, propylène, isopropylène, isopropylidène et X symbolise un atome d'halogène, notamment un atome de chlore ou de brome.

– R représente $R_3$ l'un des radicaux plus complexes suivants:

. un radical

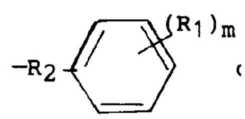

dans lequel $R_1$ et $R_2$ ont la signification donnée précédemment et m est un nombre entier de 0 à 3,

. un radical $-R_2-A-R_4$ dans lequel $R_2$ a la signification donnée précédemment, $R_4$ représente un radical alkyle linéaire ou ramifiée ayant de 1 à 6 atomes de carbone, de préférence, de 1 à 4 atomes de carbone ou un radical

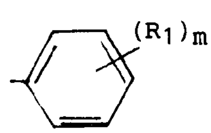

et A symbolise l'un des groupes suivants:

$-O-$, $-CO-$, $-COO-$,

$-\underset{R_5}{\underset{|}{N}}-$, $-CO-\underset{R_5}{\underset{|}{N}}-$, $-N=N-$

$-S-$, $-SO_2-$,

dans ces formules $R_5$ représente un atome d'hydrogène ou un radical alkyle linéaire ou ramifié ayant de 1 à 4 atomes de carbone, de préférence, un radical méthyle ou éthyle.

Lorsque n est supérieur à 1, les radicaux R peuvent être identiques ou différents et 2 atomes de carbone successifs du cycle benzénique peuvent être reliés entre eux par un pont cétalique tels que les radicaux méthylène dioxy ou éthylène dioxy extranucléaires.

De préférence, n est égal à (0)-1-2 ou 3.

Parmi tous les radicaux Q précités, on met en oeuvre tout préférentiellement dans le procédé de l'invention, les trihalogénométhylcarbinols répondant à la formule générale (I) dans laquelle Q représente un radical aryle répondant à la formule générale (II) dans laquelle :

– n est égal à 0, 1, 2 ou 3,
– R représente l'un des groupes de fonction suivants:
  . un radical alkyle linéaire ou ramifié, ayant de 1 à 4 atomes de carbone,
  . un radical alkoxy linéaire ou ramifié ayant de 1 à 4 atomes de carbone,
  . un radical méthylène ou éthylène dioxy,
  . un groupe -OH,
  . un groupe -CHO,
  . un radical phényle ou benzyle,
  . un atome d'halogène.

A titre d'exemples de trihalogénométhylcarbinols répondant à la formule générale (I) dans laquelle Q représente un radical aryle de formule générale (II), on peut citer : l'hydroxy-2 phényl-1 trichlorométhylcarbinol, l'hydroxy-3 phényl-1 trichlorométhylcarbinol, l'hydroxy-4 phényl-1 trichlorométhylcarbinol, l'hydroxy-2 méthyl-3 phényl-1 trichlorométhylcarbinol, l'hydroxy-2 méthyl-4 phényl-1 trichlorométhylcarbinol, l'hydroxy-2 méthyl-5 phényl-1 trichlorométhylcarbinol, l'hydroxy-3 méthyl-4 phényl-1 trichlorométhylcarbinol, le méthoxy-2 phényl-1 trichlorométhylcarbinol, le méthoxy-3 phényl-1 trichlorométhylcarbinol, le méthoxy-4 phényl-1 trichlorométhylcarbinol, le méthoxy-5 phényl-1 trichlorométhylcarbinol, l'hydroxy-3 méthoxy-4 phényl-1 trichlorométhylcarbinol, l'hydroxy-4 méthoxy-3 phényl-1 trichlorométhylcarbinol, l'hydroxy-3 diméthoxy-4,5 phényl-1 trichlorométhylcarbinol, l'hydroxy-4 diméthoxy-3,5 phényl-1 trichlorométhylcarbinol, l'hydroxy-5 phényl-1,3 bis(trichlorométhylcarbinol), l'hydroxy-2 amino-3 phényl-1 trichlorométhylcarbinol, l'hydroxy-2 amino-4 phényl-1 trichlorométhylcarbinol, l'hydroxy-2 amino-5 phényl-1 trichlorométhylcarbinol, l'hydroxy-3 amino-2 phényl-1 trichlorométhylcarbinol, l'hydroxy-2 nitro-3 phényl-1 trichlorométhylcarbinol, l'hydroxy-3 nitro-4 phényl-1 trichlorométhylcarbinol, l'hydroxy-4 nitro-3 phényl-1 trichlorométhylcarbinol, l'hydroxy-3 méthyl-4 nitro-2 phényl-1 trichlorométhylcarbinol, l'hydroxy-2 diiodo-3,5 phényl-1 trichlorométhylcarbinol, le dihydroxy-2,3 phényl-1 trichlorométhylcarbinol, le dihydroxy-2,4 phényl-1 trichlorométhylcarbinol, le dihydroxy-2,5 phényl-1 trichlorométhylcarbinol, le dihydroxy-2,6 phényl-1 trichlorométhylcarbinol, le dihydroxy-3,4 phényl-1 trichlorométhylcarbinol, le dihydroxy-3,5 phényl-1 trichlorométhylcarbinol, le dihydroxy-3,5 méthyl-4 phényl-1 trichlorométhylcarbinol, le trihydroxy-2,3,4 phényl-1 trichlorométhylcarbinol, le trihydroxy-2,4,6 phényl-1 trichlorométhylcarbinol, le trihydroxy-3,4,5 phényl-1 trichlorométhylcarbinol.

Dans la formule générale (I) des trihalogénométhylcarbinols, Q peut représenter un radical carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué par 1 à 5 radicaux $R_1$ , de préférence 1 à 3, $R_1$ ayant les significations énoncées précédemment pour les substituants du radical aryle de formule générale (II).

Comme exemples préférés de radicaux Q, on peut citer les radicaux cyclohexyle ou cyclohexène-yle, éventuellement substitué par des radicaux alkyle linéaires ou ramifiés, ayant de 1 à 4 atomes de carbone.

A titre d'exemples de trihalogénométhylcarbinols de formule (I) dans laquelle Q est un radical cycloaliphatique, on peut citer notamment le (trichlorométhylcarbinol)-1 cyclohexène-1, le (trichlorométhylcarbinol)-1 cyclohexane, le méthyl-1 (trichlorométhylcarbinol)-2 cyclohexène-1, le méthyl-1 (trichlorométhylcarbinol)-2 cyclohexane, le méthyl-1 isopropyl-4 (trichlorométhylcarbinol)-2 cyclohexène-1, le méthyl-1 isopropyl-4 (trichlorométhylcarbinol)-2 cyclohexane.

Comme mentionné précédemment, Q peut représenter un radical aliphatique acyclique, saturé ou insaturé, linéaire ou ramifié.

Plus précisément, Q représente un radical alkyle, alcényle, alcadiényle, alcynyle, linéaire ou ramifié ayant de préférence de 1 à 12 atomes de carbone.

La chaîne hydrocarbonée peut être éventuellement:

– interrompue par un des groupes suivants:

—O—, —CO—, —COO—,

-N—, —CO—N—, —N=N—,

|     |

$R_6$     $R_6$

-S—, —$SO_2$—,

dans ces formules $R_6$ représente l'hydrogène ou un radical alkyle linéaire ou ramifié ayant de 1 à 4 atomes de carbone, de préférence, un radical méthyle ou éthyle,

– et/ou porteuse de l'un des substituants suivants:

-OH, -COOH, -CHO, $NO_2$, -CN, -$NH_2$ , -SH, -X, -$CF_3$

Le radical aliphatique acyclique, saturé ou insaturé, linéaire ou ramifié peut être éventuellement porteur d'un substituant cyclique. Par cycle, on entend un cycle carbocyclique ou hétérocyclique, saturé, insaturé ou aromatique.

Le radical aliphatique acyclique peut être relié au cycle par un lien valentiel ou par l'un des groupes suivants :

—O—, —CO—, —COO—,

-N—, —CO—N—, —N=N—,

|     |

$R_6$     $R_6$

-S—, —$SO_2$—,

dans ces formules, $R_6$ ayant la signification donnée précédemment.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-même éventuellement porteurs de 1, 2, 3, 4 ou 5 radicaux $R_1$ , identiques ou différents, $R_1$ ayant la signification donnée précédemment.

A titre d'exemples de trihalogénométhylcarbinols de formule (I) dans laquelle Q représente un radical aliphatique, on peut citer notamment : le nitro-3, trichloro-1,1,1 butanol-2, le trifluoro-1,1,1 pentanol-2, le nitro-3, trichloro-1,1,1 pentanol-2, le méthyl-4 trichloro-1,1,1 pentanol-2, le trifluoro-1,1,1 hexanol-2, le diméthyl-3,3 trifluoro-1,1,1 butanol-2, l'hydroxy-2, méthoxy-4 trichloro-1,1,1 pentanol-5, le trichloro-1,1,1 heptanol-2, l'hydroxy-5 méthyl-4 trichloro-6,6,6 hexanone-3, l'hydroxy-2 trichloro-1,1,1 octanone-4, l'hydroxy-2 méthyl-6 trichloro-1,1,1 heptanone-4, l'éthyl-4 trichloro-1,1,1 hexanol-2, l'éthyl-3 trichloro-1,1,1 heptanol-2, l'hydroxy-2, trichloro-1,1,1 nonanone-4, l'hydroxy-2 méthyl-7 trichloro-1,1,1 octanone-4, le trichloro-1,1,1 triméthyl-4,6,6 heptanol-2 le trichloro-1,1,1 nonanol-2, l'hydroxy-2 trichloro-1,1,1 décanone-4, l'hydroxy-2 trichloro-1,1,1 undécanone-4, le trichloro-1,1,1 dodécanol-2 ; le trichloro-1,1,1 butène-3 ol-2, le trichloro-1,1,1 pentène-3 ol-2, le méthyl-3 trichloro-1,1,1 butène-3 ol-2, le trichloro-5,5,5 pentène-1 ol-4, le méthyl-4 trichloro-1,1,1 pentène-3 ol-2, le méthylène-4 trichloro-1,1,1 pentanol-2, le méthyl-3 trichloro-1,1,1 pentène -3 ol-2, le méthyl-4 trifluoro-1,1,1 pentène-3 ol-2, le diméthyl-3,4 trichloro-1,1,1 pentène-3 ol-2, l'éthyl-4 trichloro-1,1,1 hexène-3 ol-2, le trichloro-1,1,1 nonène-3 ol-2, le tétrachloro-1,1,1,4 nonène-3 ol-2, le trichloro-1,1,1 dodécène-3 ol-2 ; le bromo-7 trichloro-1,1,1 octène-7 yne-3 ol-2, le bromo-8 trichloro-1,1,1 octène-7 yne-3 ol-2, le trichloro-1,1,1 nonyne-3 ol-2, le trichloro-1,1,1 décyne-3 ol-2 ; le trichloro-1,1,1 (méthyl-4 cyclohexène-3 yl-1)-4 pentène -3 ol-2 ; le trichloroéthylol-9 limonène, le (tétrahydro-3,4,5,6) nonatolyl-4 trichloro-1,1,1 pentanol-2, le trichloroéthylol-9 paramenthane, le phényl-4 trichloro-1,1,1 pentène-3 ol-2, le phényl-4 trichloro-1,1,1 pentanol-2, le triméthyl-4,6,6 trichloro-1,1,1, heptène-3 ol-2, le triméthyl-4,6,6 trichloro-1,1,1 heptanol-2, le méthyl-5 trichloro-1,1,1

hexène-5 ol-2, le méthyl-5 trichloro-1,1,1 hexanol-2, le diméthyl-3,4 trichloro-1,1,1, pentanol-2, la dihydroxy-4,6 nitro-5 (trichloro-3,3,3 hydroxy-2 propyl)-2 pyrimidine.

Q peut également représenter un radical hétérocyclique monovalent, saturé ou non, comportant notamment 5 ou 6 atomes dans le cycle dont 1 ou 2 hétéroatomes tels que les atomes d'azote, de soufre et d'oxygène, les atomes de carbone de l'hétérocycle pouvant éventuellement être substitués, dans leur totalité ou pour une partie d'entre eux seulement par des radicaux $R_1$ , $R_1$ ayant la signification donnée précédemment pour les substituants du radical aryle de formule (II).

Q peut aussi représenter un radical hétérocyclique polycyclique défini comme étant soit un radical constitué par au moins 2 hétérocycles aromatique ou non contenant au moins un hétéroatome dans chaque cycle et formant entre eux des systèmes ortho- ou ortho- et péricondensés ou soit un radical constitué par au moins un cycle hydrocarboné aromatique ou non et au moins un hétérocycle aromatique ou non formant entre eux des systèmes ortho- ou ortho- et péricondensés.

A titre d'exemples de trihalogénométhylcarbinols de formule (I) dans laquelle Q représente un radical hétérocyclique, on peut citer le furyl-2 trichlorométhylcarbinol, le nitrofuryl-2 trichlorométhylcarbinol, le (méthylfuryl-5)-1 trichlorométhylcarbinol, le (N,N-diéthylfuramide-5)-1 trichlorométhylcarbinol, l'amino-2 hydroxy-4 méthyl-6 (trichlorométhylcarbinol)-5 pyrimidine, l'hydroxy-4 méthyl-6 méthylamino-2 (trichloro-2,2,2 hydroxy-1 éthyl)-5 pyrimidine, la diméthylamino-2 hydroxy-4 méthyl-6 (trichloro-2,2,2 hydroxy-1 éthyl)-5 pyrimidine.

Parmi tous les trihalogénométhylcarbinols cités précédemment à titre illustratif et sans caractère limitatif, le procédé de l'invention s'applique particulièrement bien aux composés suivants :

– le méthoxy-4 phényltrichlorométhylcarbinol
– le diméthoxy-3,4 phényltrichlorométhylcarbinol
– le diméthoxy-2,5 phényltrichlorométhylcarbinol
– le méthoxy-3 hydroxy-4 phényltrichlorométhylcarbinol
– le méthoxy-5 hydroxy-2 phényltrichlorométhylcarbinol
– l'hydroxy-4 phényltrichlorométhylcarbinol
– le dihydroxy-2,5 phényltrichlorométhylcarbinol
– le dihydroxy-3,4 phényltrichlorométhylcarbinol
– le méthylènedioxy-3,4 phényltrichlorométhylcarbinol
– le tert-butyl-4 phényltrichlorométhylcarbinol
– l'isopropyl-4 phényltrichlorométhylcarbinol
– le di-tert-butyl-3,5 phényltrichlorométhylcarbinol
– le méthyl-4 trichloro-1,1,1 pentène-3 ol-2
– le méthyl-4 trichloro-1,1,1 pentène-4 ol-2

Les composés trihalogénométhylcarbinols pouvant être soumis à la scission en aldéhydes selon le procédé de l'invention sont obtenus par des procédés décrits dans la littérature. Notamment, on peut les préparer par l'un ou l'autre des modes de préparation cités par J.H.T. LEDRUT et G. COMBES dans "Industrie chimique belge" n° 6 (1962) p. 635 à 652.

Parmi ces divers procédés d'obtention des trihalogénométhylcarbinols, certains sont plus adaptés que d'autres à la préparation de certaines familles de ce type de composés.

Ainsi, par exemple, les composés présentant un hydrogène mobile peuvent réagir avec le chloral (ou le bromal) pour donner le trihalogénométhylcarbinol correspondant.

On peut utiliser un catalyseur acide comme le chlorure d'aluminium pour faire réagir des hydrocarbures aromatiques tels que le vératrole (ou diméthoxy-1,2 benzène) avec le chloral. Pour ce type de préparation, on peut se référer, outre l'article précédemment cité, à l'article de R. QUELET dans le Bulletin de la Société Chimique de France (1954), p. 932.

En partant de phénols, on peut faire réagir le chloral en présence de carbonate de potassium anhydre [voir M. PAULY Berichte der Deutschen Gesellschaft 56, 979 (1923)].

Conformément au procédé de l'invention, on effectue la scission du trihalogénométhylcarbinol en aldéhyde correspondant, dans un solvant organique en présence d'un sel à propriétés basiques.

Intervient donc, dans le procédé de l'invention, à titre de catalyseur, un sel à propriétés basiques.
Un premier impératif qui préside au choix dudit sel est qu'il doit être insoluble dans le milieu réactionnel.
Une autre caractéristique de ce sel est que son anion présente un $pk_a$ défini dans les intervalles précédemment définis.

Conviennent particulièrement bien à la mise en oeuvre du procédé de l'invention, les sels de métaux alcalins, de métaux alcalino-terreux et/ou les métaux de transition du groupe VIII de la classification périodique.

Par sels de métaux alcalins, on entend dans le présent texte, les sels de lithium, de sodium, de potassium, de rubidium et de césium. Parmi tous ces sels, on préfère utiliser les sels de césium et de potassium.

Par sels de métaux alcalino-terreux, on entend dans le présent texte, les sels de béryllium, de magnésium,

de calcium, de strontium et de baryum. On préfère, le plus souvent, utiliser les sels de baryum.

En ce qui concerne les sels de métaux de transition, on peut faire appel à des sels de métaux divalents, trivalents et tétravalents et l'on peut citer notamment, les sels de métaux divalents tels que le manganèse, le cobalt, le nickel, le cuivre, le zinc ; les sels de métaux trivalents tels que le fer, le chrome, l'aluminium, les métaux de terres rares à savoir l'yttrium et les éléments métalliques ayant un numéro atomique de 57 à 71 et le scandium. Parmi les différents sels précités, on choisit préférentiellement les sels de lanthane et de zinc.

Pour ce qui est de la forme sous laquelle se trouve le ou les éléments métalliques précités, on peut citer plus spécialement les formes suivantes : carbonates, hydrogénocarbonates, fluorures, phosphates, hydrogénophosphates, pyrophosphates, sulfates, hydrogénosulfates, sulfites, hydrogénosulfites, borates, hydrogénoborates.

A titre de sels basiques préférés, on choisit le carbonate de césium.

Les différents sels peuvent être mis en oeuvre tels quels ou bien sous une forme supportée, par exemple sur des oxydes minéraux comme l'alumine, la silice, les argiles naturelles ou synthétiques, etc...

Les sels mis en oeuvre sont indifféremment sous forme anhydre ou hydratée mais l'on préfère la forme hydratée.

Selon le procédé de l'invention, la scission du trihalogénométhylcarbinol en aldéhyde correspondant est effectué dans un solvant organique qui est un solvant organique aprotique ayant une polarité faible ou moyenne. On le choisit de telle sorte qu'il est une constante diélectrique mesurée entre 20°C et 25°C ,supérieure à 0 et inférieure à 20, de préférence comprise entre 5 et 15.

On peut mettre en oeuvre selon l'invention, tout solvant organique liquide dans les conditions réactionnelles, répondant aux caractéristiques précitées et solubilisant le trihalogénométhylcarbinol.

Afin de déterminer si un solvant organique convient dans la mise en oeuvre du procédé de l'invention, on peut se reporter aux tables de l'ouvrage : Techniques of Chemistry, II - Organic solvents - p. 536 et suivantes, 3ème édition (1970).

Comme exemples de solvants aprotiques peu ou moyennement polaires susceptibles d'être mis en oeuvre dans le procédé de l'invention, on peut citer les hydrocarbures aliphatiques ou aromatiques, halogénés ou non.

A titre d'hydrocarbures aliphatiques ou aromatiques, on peut citer l'hexane, l'heptane, l'octane, le nonane, le décane ou le cyclohexane, le toluène, le xylène.

A titre d'hydrocarbures halogénés aliphatiques ou aromatiques, on peut mentionner : les hydrocarbures perchlorés tels que notamment le tétrachlorure de carbone, le tétrachloroéthylène, l'hexachloroéthane, l'hexachloropropène et l'hexachlorobutadiène, les hydrocarbures partiellement chlorés tels que le dichlorométhane, le chloroforme, le dichloro-1,2 éthane, le trichloro-1,1,1 éthane, le tétrachloro-1,1,2,2 éthane, le pentachloroéthane, le trichloroéthylène, le chloro-1 butane, le dichloro-1,2 butane ; le monochlorobenzène, le dichloro-1,2 benzène, le dichloro-1,3 benzène, le dichloro-1,4 benzène , le trichloro-1,2,4 benzène ou des mélanges de différents chlorobenzènes ; le bromoforme, le bromoéthane ou le dibromo-1,2 éthane ; le monobromobenzène ou des mélanges de monobromobenzène avec un ou plusieurs dibromobenzènes ; le bromo-1 naphtalène.

On peut également faire appel à des solvants tels que les phénols comme le phénol, l'o-crésol, le m-crésol ou le p-crésol ou les nitriles alphatiques ou aromatiques comme l'hexanenitrile, l'octanenitrile, le benzonitrile.

Une autre classe de solvants convenant également à l'invention, sont les éther-oxydes aliphatiques ou aromatiques et, plus particulièrement, le diméthyléther de l'éthylèneglycol (ou diméthoxy-1,2 éthane), le diméthyléther du diéthylèneglycol (ou diméthoxy-1,5 oxa-3 pentane), le diméthoxy-1,8 dioxa-3,6 octane, le diméthoxy-1,11 trioxa-3,6,9 undécane, le propyléther, l'isopropyléther, le butyléther, le méthyl-tertiobutyléther, le pentyléther, l'isopentyléther, le phényléther, le benzyléther, le dioxane, l'anisole, le phénétole, le p-diméthoxy-benzène, le vératrole.

Conviennent également à la mise en oeuvre du procédé de l'invention, les solvants tels que les esters phosphoriques comme, par exemple, le butylphosphate, le tributylphosphate, etc...

Parmi tous les solvants précités, on choisit plus préférentiellement, le dichloro-1,2 benzène.

On peut également utiliser un mélange de solvants.

Conformément au procédé de l'invention, la réaction de scission de trihalogénométhylcarbinols en aldéhydes correspondants est effectuée en présence d'un catalyseur à savoir un sel à propriétés basiques, dans un solvant organique aprotique peu ou moyennement polaire, les différents composés étant mis en oeuvre dans les proportions définies ci-après.

La concentration du trihalogénométhylcarbinol dans le solvant organique n'est pas critique et peut varier dans de larges limites, par exemple de 0,1 à 10 mol/litre. Toutefois, une concentration choisie entre 0,5 et 2 mol/litre est préférée.

En ce qui concerne la quantité de catalyseur mis en jeu exprimée par rapport au trihalogénométhylcarbinol, elle est telle que le rapport pondéral sel basique/trihalogénométhylcarbinol varie de 0,01 à 1,0, de préférence de 0,30 à 0,50.

Une variante préférée du procédé de l'invention consiste à effectuer la réaction de scission des trihalogénocarbinols en présence d'une très faible quantité d'eau.

En effet, on a constaté de manière inattendue, que la présence d'une quantité d'eau exprimée par le rapport entre le nombre de oles de catalyseur et le nombre de moles d'eau comprise entre 0,5 et 10,0, et de préférence entre 2,0 et 8,0, permettait non seulement d'avoir une très bonne sélectivité de la réaction mais également un taux de conversion supérieur.

Ainsi, l'eau peut être apportée sous n'importe quelle forme, par exemple, par le catalyseur lorsqu'il s'agit d'un sel sous une forme hydratée ou par voie extérieure, par addition de la quantité d'eau désirée.

La température de la réaction peut être comprise entre 25°C et 200°C et, de préférence, entre 80°C et 170°C.

La réaction peut être conduite sous pression atmosphérique ou bien sous pression réduite comprise, de préférence, entre 10 et 500 millibars.

On préfère conduire la réaction sous pression atmosphérique mais sous atmosphère contrôlée de gaz inertes tels que l'azote ou les gaz rares, par exemple l'argon.

La durée de la réaction peut être très variable. Elle dépend entre autres de la nature du trihalogénométhylcarbinol, de son taux de conversion et de la température réactionnelle. Elle se situe, le plus souvent, entre 15 minutes et 10 heures, de préférence entre 30 minutes et 5 heures.

D'un point de vue pratique, la réaction est aisément réalisée en chargeant dans l'appareillage le catalyseur, le solvant organique, le trihalogénométhylcarbinol puis en portant le mélange réactionnel sous agitation à la température désirée, éventuellement sous pression réduite, pendant la durée nécessaire à l'achèvement de la réaction.

L'ordre d'introduction des réactifs n'est pas critique mais celui précédemment indiqué est préféré.

Généralement, on élimine au fur et à mesure de sa formation, le trihalogénométhane formé, par distillation en continu.

En fin de réaction, le mélange réactionnel comprend l'aldéhyde formé, le trihalogénométhylcarbinol n'ayant pas réagi en faibles quantités, le catalyseur et le solvant réactionnel.

Le milieu réactionnel se trouve sous la forme d'une solution limpide dans laquelle le catalyseur se trouve en suspension. Il est ainsi particulièrement aisé de récupérer le catalyseur selon les techniques classiques de séparation solide/liquide telles que décantation, filtration, essorage ou centrifugation.

On récupère l'aldéhyde formé, le plus souvent par distillation sous pression atmosphérique ou sous pression réduite (10 à 500 millibars) si l'aldéhyde est un aldéhyde lourd à haut point d'ébullition (par exemple supérieur ou égal à 150°C).

On donne, ci-après, des exemples de réalisation de l'invention.

Les exemples suivants sont donnés, à titre illustratif, sans caractère limitatif.

Dans les exemples, les abréviations suivantes signifient:

$$\text{Conversion} = \frac{\text{nombre de moles de trihalogénométhylcarbinol transformées}}{\text{nombre de moles de trihalogénométhylcarbinol introduites}}\ \%$$

$$\text{Rendement} = \frac{\text{nombre de moles d'aldéhyde formées}}{\text{nombre de moles de trihalogénométhylcarbinol introduites}}\ \%$$

$$\text{Sélectivité} = \frac{\text{nombre de moles d'aldéhyde formées}}{\text{nombre de moles de trihalogénométhylcarbinol transformées}}\ \%$$

EXEMPLE 1

Dans un ballon tricol en verre de 50 $cm^3$ équipé d'une agitation magnétique, d'un système de distillation, d'une arrivée de gaz inerte (argon), d'un thermomètre et d'un dispositif de chauffage par bain d'huile, on charge :

– 1 g (4 mmol) de méthoxy-4 phényl-1 trichlorométhylcarbinol, de formule :

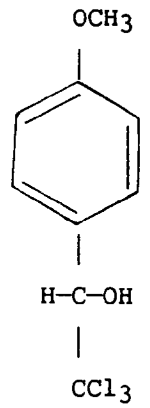

– 6,5 g de dichloro-1,2 benzène

Sous atmosphère de gaz inerte et à température ambiante (25°C), on maintient le milieu réactionnel sous agitation jusqu'à dissolution complète du méthoxy-4 phényl-1 trichlorométhylcarbinol.

On charge ensuite 1 g (3 mmol) de carbonate de césium.

On chauffe à 90°C dans la masse pendant 4 heures.

On distille le chloroforme au fur et à mesure de la réaction.

On laisse refroidir ensuite jusqu'à 25°C et l'on sépare le catalyseur par filtration sur verre fritté n° 3.

On analyse la phase organique par chromatographie liquide haute performance (CHLP).

On a obtenu 4,00 mmol de méthoxy-4 benzaldéhyde (anisaldéhyde).

On a déterminé une conversion du méthoxy-4 phényl-1 trichlorométhylcarbinol de 100 % et une sélectivité en anisaldéhyde de 100 %.

EXEMPLES 2 A 9

On reproduit l'exemple 1 à la seule différence que l'on change la nature du catalyseur mis en jeu :

– Exemple 2 : fluorure de césium CsF
– Exemple 3 : hydrogénocarbonate de césium $CsHCO_3$
– Exemple 4 : carbonate de césium $Cs_2CO_3$
– Exemple 5 : hydrogénophosphate de césium $Cs_2HPO_4$
– Exemple 6 : fluorure de potassium déposé sur alumine $KF/Al_2O_3$
– Exemple 7 : carbonate de potassium $K_2CO_3$
– Exemple 8 : carbonate de potassium $K_2CO_3$

Dans les exemples 7 et 8, la masse réactionnelle est chauffée à 130°C respectivement pendant 4 heures et 18 heures.

Les résultats obtenus sont consignés dans le tableau I.

Tableau I

| Réf. de l'exemple | Catalyseur | Nombre de mmol de catalyseur | Conversion % | Rendement % | Sélectivité % |
|---|---|---|---|---|---|
| 2 | CsF | 6,6 | 100 | 100 | 100 |
| 3 | $CsHCO_3$ | 5,2 | 47 | 47 | 100 |
| 4 | $Cs_2CO_3$ | 1,5 | 95 | 95 | 100 |
| 5 | $Cs_2HPO_4$ | 2,8 | 61 | 61 | 100 |
| 6 | $KF/Al_2O_3$ | 6,9 | 72 | 67 | 93 |
| 7 | $K_2CO_3$ | 7,3 | 72 | 72 | 100 |
| 8 | $K_2CO_3$ | 7,3 | 100 | 100 | 100 |

EXEMPLE 9

On reproduit l'exemple 1 à la différence près que l'on remplace le dichloro-1,2 benzène par de l'anisole anhydre.

On met en oeuvre 5,2 g d'anisole anhydre qui ont été préalablement séchés par passage sur tamis moléculaire 0,3 nm, commercialisé par la Société PROLABO.

On détermine par CLHP, 4 mmol d'anisaldéhyde, soit un rendement de 100 %.

EXEMPLE 10

On répète l'exemple 1, mais l'on charge 100 mg (0,3 mmol) de carbonate de césium préalablement calciné pendant 1 heure à 200°C sous $4.10^{-2}$ mm de mercure.

On a également séché le dichloro-1,2 benzène sur tamis moléculaire 0,3 nm.

On détermine par CLHP, 1,48 mmol d'anisaldéhyde et 2,48 mmol de méthoxy-4 phényl-1 trichlorométhylcarbinol non transformé, ce qui correspond à une conversion de 38 % et une sélectivité de 97 %.

ESSAI COMPARATIF a

On reproduit l'exemple 1, mais l'on ne charge pas le carbonate de césium.

On agite le milieu réactionnel et l'on chauffe 4 heures à 90°C.

En l'absence de sel à propriétés basiques, il n'y a pas de réaction.

EXEMPLES 11 A 15

On reproduit l'exemple 10 à la seule différence que l'on charge dans le milieu réactionnel x $cm^3$ d'eau mesurés précisément, de manière à fixer le rapport r défini comme étant le rapport entre le nombre de moles de catalyseur et le nombre de moles d'eau.

Tableau II

| Réf. de l'exemple | x | r | Conversion % | Rendement % | Sélectivité % |
|---|---|---|---|---|---|
| 11 | 1 | 5,5 | 61 | 60 | 98 |
| 12 | 2 | 3 | 62 | 60 | 97 |
| 13 | 5 | 1 | 38 | 38 | 100 |
| 14 | 10 | 0,6 | 24 | 24 | 100 |
| 15 | 20 | 0,3 | 24 | 24 | 100 |

On note l'influence bénéfique de la présence d'eau sur la conversion du méthoxy-4 phényl-1 trichlorométhylcarbinol.

EXEMPLE 16

Dans un ballon tricol en verre de 50 cm³ équipé d'une agitation magnétique, d'un système de distillation, d'une arrivée de gaz inerte (argon), d'un thermomètre et d'un dispositif de chauffage par bain d'huile, on charge :

– 0,80 g (3 mmol) d'hydroxy-4 phényl-1 trichlorométhylcarbinol, de formule

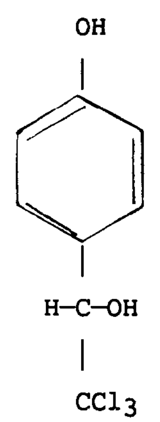

– 4,80 g (5 ml) de diméthyléther de diéthylèneglycol

Sous atmosphère de gaz inerte et à température ambiante, on maintient le milieu réactionnel sous agitation jusqu'à dissolution de l'hydroxy-4 phényl-1 trichlorométhylcarbinol.

On charge ensuite 1 g (6,6 mmol) de fluorure de césium.

On chauffe à 100°C dans la masse pendant 4 heures.

On distille le chloroforme co-produit au fur et à mesure de la réaction.

On laisse refroidir et l'on filtre le catalyseur sur verre fritté n° 3.

On analyse la phase organique par chromatographie liquide haute performance.

On a obtenu 3,7 mmol d'hydroxy-4 benzaldéhyde.

On a déterminé un taux de conversion d'hydroxy-4 phényl-1 trichlorométhylcarbinol de 100 % et une sélectivité en hydroxy-4 benzaldéhyde de 56 %.

EXEMPLES 17 à 20

On reproduit l'exemple 1 à la seule différence que l'on modifie la nature du catalyseur mis en jeu :

– Exemple 17 : carbonate de césium $Cs_2CO_3$

- Exemple 18 : hydrogénocarbonate de césium $CsHCO_3$
- Exemple 19 : hydrogénophosphate de césium $Cs_2HPO_4$
- Exemple 20 : fluorure de potassium déposé sur alumine $KF/Al_2O_3$

Les résultats obtenus sont consignés dans le tableau III.

Tableau III

| Réf. de l'exemple | Catalyseur | mmol | Conversion % | Sélectivité % | Rendement % |
|---|---|---|---|---|---|
| 17 | $Cs_2CO_3$ | 6,1 | 100 | 22 | 22 |
| 18 | $CsHCO_3$ | 6,5 | 100 | 53 | 53 |
| 19 | $Cs_2HPO_4$ | 7,2 | 100 | 54 | 54 |
| 20 | $KF/Al_2O_3$ | 6,4 | 90 | 40 | 36 |

EXEMPLE 21

On a reproduit l'exemple 16, aux différences près:
- on charge 4,9 g de tributylphosphate à la place de 4,8 g de diméthyléther de diéthylène glycol,
- la durée de chauffage n'est que de 30 minutes.

On a déterminé un taux de conversion de l'hydroxy-4 phényl-1 trichlorométhylcarbinol de 74 %, une sélectivité en hydroxy-4 benzaldéhyde de 54 % et un rendement de 40 %.

EXEMPLE 22

Dans un réacteur de 30 $cm^3$ tel que décrit dans l'exemple 16 mais alimenté par un courant d'azote au lieu d'un courant d'argon, on charge :
- 1 g (5 mmol) de méthyl-4 trichloro-1,1,1 pentène-4 ol-2,
- 13 g (10 mol) de dichloro-1,2 benzène,
- 1 g (3 mmol) de carbonate de césium $CsCO_3$.

On chauffe jusqu'à obtention d'une température de 160°C que l'on maintient pendant 1 heure.

On entraîne par un courant d'azote, le chloroforme, co-produit de la réaction et le méthyl-3 buténal-2 que l'on piège à - 40°C par un mélange d'acétonitrile et de carboglace.

On laisse refroidir ensuite jusqu'à environ 25°C et l'on sépare le catalyseur par filtration sur verre fritté n°4.

On analyse la phase organique par chromatographie en phase gazeuse.

On a obtenu 1,5 mmol de méthyl-3 buténal-2 et 1,5 mmol de chloroforme.

On a déterminé un taux de conversion du méthyl-4 trichloro-1,1,1 pentène-4 ol-2 de 94 % et une sélectivité en méthyl-3 buténal-2 de 32 %.

**Revendications**

**1 -** Procédé de préparation d'un aldéhyde par scission de l'alcool secondaire α-trihalogéné correspondant caractérisé par le fait que la réaction est effectuée dans un solvant aprotique peu ou moyennement polaire et en présence d'un catalyseur qui est un sel à propriétés basiques, insoluble dans le milieu réactionnel.

**2 -** Procédé selon la revendication 1 caractérisé par le fait que l'alcool secondaire α-trihalogéné répond à la formule générale (I) :

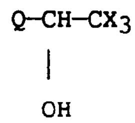

(I)

dans ladite formule, Q représente un radical hydrocarboné monovalent, éventuellement substitué ayant de 1 à 40 atomes de carbone et X symbolise un atome d'halogène, chlore, fluor, brome, iode et, de préférence, le chlore.

**3 -** Procédé selon l'une des revendications 1 et 2 caractérisé par le fait que l'alcool secondaire α-trihalogéné répond à la formule générale (I) dans laquelle Q représente un radical hydrocarboné monovalent, substitué ou non qui peut être un radical aliphatique acyclique saturé ou insaturé, linéaire ou ramifié ; un radical carboxycyclique ou hétérocyclique saturé, insaturé ou aromatique, monocyclique ou polycyclique.

**4 -** Procédé selon l'une des revendications 1 à 3 caractérisé par le fait que l'alcool secondaire α-trihalogéné répond à la formule générale (I) dans laquelle Q représente un radical aryle répondant à la formule générale (II)

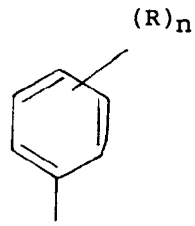

(II)

dans ladite formule (II):

– n est un nombre entier de 0 à 5, de préférence de 0 à 3,

– R représente $R_1$, l'un des groupes ou fonctions suivantes:

. un radical alkyle, linéaire ou ramifié, ayant de 1 à 6 atomes de carbone, de préférence de 1 à 4 atomes de carbone,

. un radical alcényle linéaire ou ramifié ayant de 2 à 6 atomes de carbone, de préférence, de 2 à 4 atomes de carbone,

. un radical alkoxy linéaire ou ramifié ayant de 1 à 6 atomes de carbone, de préférence de 1 à 4 atomes de carbone,

. un radical de formule

$-R_2-OH$
$-R_2-COOH$
$-R_2-CHO$
$-R_2-NO_2$
$-R_2-CN$
$-R_2-NH_2$
$-R_2-SH$
$-R_2-X$
$-R_2-CF_3$

dans lesdites formules $R_2$ représente un lien valentiel ou un radical hydrocarboné divalent, linéaire ou ramifié, saturé ou insaturé, ayant de 1 à 4 atomes de carbone et X symbolise un atome d'halogène, notamment un atome de chlore ou de brome.

– R représente $R_3$ l'un des radicaux plus complexes suivants:

. un radical

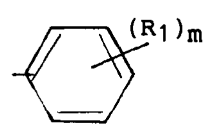

dans lequel $R_1$ et $R_2$ ont la signification donnée précédemment et m est un nombre entier de 0 à 3,

. un radical $-R_2-A-R_4$ dans lequel $R_2$ a la signification donnée précédemment, $R_4$ représente un radical alkyle linéaire ou ramifiée ayant de 1 à 6 atomes de carbone, de préférence, de 1 à 4 atomes de carbone ou un radical

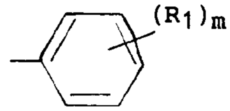

et A symbolise l'un des groupes suivants:

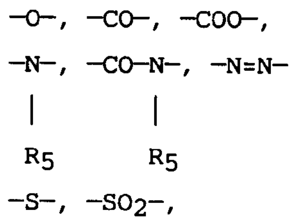

dans ces formules $R_5$ représente un atome d'hydrogène ou un radical alkyle linéaire ou ramifié ayant de 1 à 4 atomes de carbone.

**5 -** Procédé selon la revendication 4 caractérisé par le fait que l'alcool secondaire α-trihalogéné répond à la formule générale (I) dans laquelle Q représente un radical aryle répondant à la formule générale (II) dans laquelle n est supérieur à 1 et les radicaux R sont identiques ou différents et deux atomes de carbone successifs du cycle benzénique sont reliés entre eux par un pont cétalique, de préférence, par un radical méthylène ou éthylène extranucléaire.

**6 -** Procédé selon l'une des revendications 4 et 5 caractérisé par le fait que l'alcool secondaire α-trihalogéné répond à la formule générale (I) dans laquelle Q représente un radical aryle répondant à la formule générale (II) dans laquelle

– n est égal à 0, 1, 2 ou 3,

– R représente l'un des groupes de fonction suivants:

. un radical alkyle linéaire ou ramifié, ayant de 1 à 4 atomes de carbone,

. un radical alkoxy linéaire ou ramifié, ayant de 1 à 4 atomes de carbone,

. un radical méthylène ou éthylène dioxy,

. un groupe -OH,

. un groupe -CHO,

. un radical phényle ou benzyle,

. un atome d'halogène.

**7 -** Procédé selon l'une des revendications 1 à 3 caractérisé par le fait que l'alcool secondaire α-trihalogéné répond à la formule (I) dans laquelle Q représente un radical carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué par 1 à 5 radicaux $R_1$ , de préférence 1 à 3, $R_1$ ayant les significations énoncées précédemment dans la revendication 4.

**8 -** Procédé selon la revendication 7 caractérisé par le fait que l'alcool secondaire α-trihalogéné répond à la formule générale (I) dans laquelle Q représente un radical cyclohexyle ou cyclohexène-yle, éventuellement substitué par des radicaux alkyle linéaires ou ramifiés, ayant de 1 à 4 atomes de carbone.

**9 -** Procédé selon l'une des revendications 1 à 3 caractérisé par le fait que l'alcool secondaire α-trihalogéné répond à la formule générale (I) dans laquelle Q représente un radical alkyle, alcényle, alcadiényle, alcynyle, linéaire ou ramifié ayant de préférence de 1 à 12 atomes de carbone ; la chaîne hydrocarbonée pouvant être éventuellement :

– interrompue par un des groupes suivants:

−O−, −CO−, −COO−,

−N−, −CO−N−, −N=N−,

|           |

$R_6$           $R_6$

−S−, $-SO_2-$,

dans ces formules $R_6$ représente l'hydrogène ou un radical alkyle linéaire ou ramifié ayant de 1 à 4 atomes de carbone, de préférence, un radical méthyle ou éthyle,

– et/ou porteuse de l'un des substituants suivants:

-OH, -COOH, -CHO, $NO_2$, -CN, $-NH_2$, -SH, -X, $-CF_3$

**10 -** Procédé selon la revendication 9 caractérisé par le fait que l'alcool secondaire α-trihalogéné répond à la formule générale (I) dans laquelle Q représente un radical aliphatique acyclique, saturé ou insaturé, linéaire ou ramifié porteur d'un substituant cyclique, ledit radical aliphatique acyclique pouvant être relié au cycle par un lien valentiel ou par l'un des groupes suivants :

−O−, −CO−, −COO−,

−N−, −CO−N−, −N=N−,

|           |

$R_6$           $R_6$

−S−, $-SO_2-$,

dans ces formules, $R_6$ ayant la signification donnée précédemment dans la revendication 9.

**11 -** Procédé selon la revendication 10 caractérisé par le fait que l'alcool secondaire α-trihalogéné répond à la formule générale (I) dans laquelle Q représente un radical aliphatique acyclique, saturé ou insaturé, linéaire ou ramifié porteur d'un substituant cycloaliphatique, aromatique ou hétérocyclique éventuellement porteur de 1 à 5 radicaux $R_1$, identiques ou différents, tels que définis dans la revendication 4.

**12 -** Procédé selon l'une des revendications 1 à 3 caractérisé par le fait que l'alcool secondaire α-trihalogéné répond à la formule générale (I) dans laquelle Q représente un radical hétérocyclique monovalent, saturé ou non, comportant notamment 5 ou 6 atomes dans le cycle dont 1 ou 2 hétéroatomes tels que les atomes d'azote, de soufre et d'oxygène, les atomes de carbone de l'hétérocycle pouvant éventuellement être substitués, dans leur totalité ou pour une partie d'entre eux seulement par des radicaux $R_1$ , $R_1$ ayant la signification donnée précédemment dans la revendication 4.

**13 -** Procédé selon l'une des revendications 1 à 12 caractérisé par le fait que l'alcool secondaire α-trihalogéné est :

– le méthoxy-4 phényltrichlorométhylcarbinol
– le diméthoxy-3,4 phényltrichlorométhylcarbinol
– le diméthoxy-2,5 phényltrichlorométhylcarbinol
– le méthoxy-3 hydroxy-4 phényltrichlorométhylcarbinol
– le méthoxy-5 hydroxy-2 phényltrichlorométhylcarbinol
– l'hydroxy-4 phényltrichlorométhylcarbinol
– le dihydroxy-2,5 phényltrichlorométhylcarbinol
– le dihydroxy-3,4 phényltrichlorométhylcarbinol
– le méthylènedioxy-3,4 phényltrichlorométhylcarbinol
– le tert-butyl-4 phényltrichlorométhylcarbinol
– l'isopropyl-4 phényltrichlorométhylcarbinol
– le di-tert-butyl-3,5 phényltrichlorométhylcarbinol
– le méthyl-4 trichloro-1,1,1 pentène-3 ol-2
– le méthyl-4 trichloro-1,1,1 pentène-4 ol-2

**14 -** Procédé selon l'une des revendications 1 à 13 caractérisé par le fait que le catalyseur est un sel dont l'anion a un $pK_b$ en milieu aqueux à 25°C, compris entre 0 et 14, de préférence entre 0 et 11, et encore plus préférentiellement entre 0 et 8.

**15 -** Procédé selon l'une des revendications 1 à 14 caractérisé par le fait que le catalyseur est un sel de métal alcalin, de métal alcalino-terreux et/ou d'un métal de transition du groupe VIII de la classification périodique.

**16** - Procédé selon la revendication 15 caractérisé par le fait que le catalyseur est un sel de lithium, de sodium, de potassium, de rubidium et de césium, de préférence un sel de césium ou de potassium.

**17** - Procédé selon la revendication 15 caractérisé par le fait que le catalyseur est un sel de béryllium, de magnésium, de calcium, de strontium et de baryum, de préférence un sel de baryum.

**18** - Procédé selon la revendication 15 caractérisé par le fait que le catalyseur est un sel de métal divalent, trivalent ou tétravalent d'un métal de transition du groupe VIII de la classification périodique.

**19** - Procédé selon la revendication 15 caractérisé par le fait que le catalyseur est un sel de métal divalent tel que le manganèse, le cobalt, le nickel, le cuivre, le zinc ; un sel de métal trivalent tel que le fer, le chrome, l'aluminium, de métal de terre rare ou de scandium ; ledit sel est, de préférence, un sel de lanthane ou de zinc.

**20** - Procédé selon l'une des revendications 14 à 19 caractérisé par le fait que le catalyseur est un sel métallique sous les formes suivantes : carbonates, hydrogénocarbonates, fluorures, phosphates, hydrogénophosphates, pyrophosphates, sulfates, hydrogénosulfates, sulfites, hydrogénosulfites, borates, hydrogénoborates.

**21** - Procédé selon l'une des revendications 14 à 20 caractérisé par le fait que le catalyseur est le carbonate de césium, le fluorure de césium, le carbonate de potassium, le fluorure de potassium.

**22** - Procédé selon l'une des revendications 14 à 21 caractérisé par le fait que le catalyseur est un sel métallique sous forme anhydre ou hydraté, mis en oeuvre tel quel ou sous forme supportée.

**23** - Procédé selon l'une des revendications 14 à 22 caractérisé par le fait que le rapport entre le nombre de moles de sel basique et le nombre de moles d'eau est compris entre 0,5 et 10,0 et, de préférence, entre 2,0 et 8,0.

**24** - Procédé selon l'une des revendications 1 à 22 caractérisé par le fait que le solvant organique a une constante diélectrique supérieure à 0 et inférieure à 20, de préférence comprise entre 5 et 15.

**25** - Procédé selon la revendication 24 caractérisé par le fait que le solvant organique est choisi parmi les hydrocarbures aliphatiques ou aromatiques halogénés ou non ; les nitriles aliphatiques ou aromatiques ; les phénols ou les éther-oxydes aliphatiques ou aromatiques ; les esters phosphoriques.

**26** - Procédé selon l'une des revendications 24 et 25 caractérisé par le fait que le solvant organique est choisi parmi les solvants suivants : l'hexane, l'heptane, l'octane, le nonane, le décane ou le cyclohexane, le toluène, le xylène ; le tétrachlorure de carbone, le tétrachloroéthylène, l'hexachloroéthane, l'hexachloropropène et l'hexachlorobutadiène ; le dichlorométhane, le chloroforme, le dichloro-1,2 éthane, le trichloro-1,1,1 éthane, le tétrachloro-1,1,2,2 éthane, le pentachloroéthane, le trichloroéthylène, le chloro-1 butane, le dichloro-1,2 butane ; le monochlorobenzène, le dichloro-1,2 benzène, le dichloro-1,3 benzène, le dichloro-1,4 benzène , le trichloro-1,2,4 benzène ou des mélanges de différents chlorobenzènes ; le bromoforme, le bromoéthane ou le dibromo-1,2 éthane ; le monobromobenzène ou des mélanges de monobromobenzène avec un ou plusieurs dibromobenzènes ; le bromo-1 naphtalène ; le phénol, l'o-crésol, le m-crésol ou le p-crésol, l'hexanenitrile, l'octanenitrile, le benzonitrile ; le diméthyléther de l'éthylèneglycol (ou diméthoxy-1,2 éthane), le diméthyléther du diéthylèneglycol (ou diméthoxy-1,5 oxa-3 pentane), le diméthoxy-1,8 dioxa-3,6 octane, le diméthoxy-1,11 trioxa-3,6,9 undécane, le propyléther, l'isopropyléther, le butyléther, le méthyltertiobutyléther, le pentyléther, l'isopentyléther, le phényléther, le benzyléther, le dioxane, l'anisole, le phénétole, le p-diméthoxybenzène, le vératrole, le butylphosphate, le tributylphosphate.

**27** - Procédé selon l'une des revendications 24 à 26 caractérisé par le fait que le solvant organique est le dichloro-1,2 benzène ou l'anisole.

**28** - Procédé selon l'une des revendications 1 à 27 caractérisé par le fait que la concentration de l'alcool secondaire $\alpha$-trihalogéné dans le solvant organique varie entre 0,1 et 10 mol/litre, de préférence entre 0,5 et 2 mol/litre.

**29** - Procédé selon l'une des revendications 1 à 28 caractérisé par le fait que le rapport pondéral sel basique/alcool secondaire $\alpha$-trihalogéné varie de 0,01 à 1,0, de préférence de 0,03 à 0,50.

**30** - Procédé selon l'une des revendications 1 à 29 caractérisé par le fait que la température réactionnelle est comprise entre 25°C et 200°C, de préférence entre 80°C et 170°C.

Office européen des brevets

# RAPPORT DE RECHERCHE EUROPEENNE

Numero de la demande

EP 91 40 2737

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | FR-A-2 244 743 (SHELL INTERNATIONALE RESEARCH MAATSCHAPPIJ B.V.)<br>* page 2 - page 3; revendications *<br>--- | 1-6, 13-17, 20,21 | C07C45/51<br>C07C47/575<br>C07C47/565<br>C07C47/542<br>C07C47/21 |
| A | RESEARCH DISCLOSURE<br>1976,<br>page 12; 'Preparation of para-tert.Butyl Benzaldehyde'<br>* no. 14211; Résumé *<br>--- | 1-4, 14-16, 20,21 | |
| A | EP-A-0 120 966 (MITSUI TOATSU CHEMICALS, INC.)<br>* page 4 - page 5; revendications *<br>----- | 1,14-17, 20,21, 25,26 | |
| | | | **DOMAINES TECHNIQUES RECHERCHES (Int. Cl.5 )** |
| | | | C07C |

Le présent rapport a été établi pour toutes les revendications

| Lieu de la recherche | Date d'achèvement de la recherche | Examinateur |
|---|---|---|
| LA HAYE | 23 JANVIER 1992 | BONNEVALLE E.I. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)